(19) Europäisches Patentamt
European Patent Office
Office européen des brevets

(11) EP 2 375 481 B1

(12) **FASCICULE DE BREVET EUROPEEN**

(45) Date de publication et mention
de la délivrance du brevet:
**22.08.2012 Bulletin 2012/34**

(51) Int Cl.:
*H01M 4/90* (2006.01)     *H01M 8/16* (2006.01)
*C12N 9/00* (2006.01)     *C12N 9/02* (2006.01)
*C12N 9/04* (2006.01)

(21) Numéro de dépôt: **11160991.3**

(22) Date de dépôt: **04.04.2011**

(54) **Biopile à glucose**

Biobrennstoffzelle mit Glukose

Biofuel cell with glucose

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.04.2010 FR 1052657**

(43) Date de publication de la demande:
**12.10.2011 Bulletin 2011/41**

(73) Titulaire: **Université Joseph Fourier
38041 Grenoble Cedex 9 (FR)**

(72) Inventeurs:
- **Cinquin, Philippe
  38330, SAINT NAZAIRE LES EYMES (FR)**
- **Gondran, Chantal
  38000, GRENOBLE (FR)**
- **Giroud, Fabien
  38160, CHEVRIERES (FR)**
- **Cosnier, Serge
  38920, CROLLES (FR)**

(74) Mandataire: **de Beaumont, Michel et al
1bis, rue Champollion
38000 Grenoble (FR)**

(56) Documents cités:
**WO-A1-2009/136092**

- **COSNIER S ET AL: "An easy compartment-less biofuel cell construction based on the physical co-inclusion of enzyme and mediator redox within pressed graphite discs", ELECTROCHEMISTRY COMMUNICATION, vol. 12, no. 2, 1 février 2010 (2010-02-01), pages 266-269, XP026858666, ELSEVIER, AMSTERDAM, NL ISSN: 1388-2481 [extrait le 2009-12-16]**
- **N. KAKEHI AND AL: "A Novel wireless glucose sensor employing direct electron transfer principle based enzyme fuel cell", BIOSENSORS AND BIOELECTRONICS, vol. 22, 1 janvier 2007 (2007-01-01), pages 2250-2255, XP002600793, DOI: 10.1016/j.bios.2006.11.004**
- **S. COSNIER AND AL: "Carbon cavity microelectrode for electrical wiring of enzyme by insoluble electroactive species in aqueous media", ELECTROANALYSIS, vol. 20, no. 7, 1 janvier 2008 (2008-01-01), pages 750-756, XP002600794, DOI: 10.1002/elan.200704095**
- **KUWAHARA T ET AL: "Fabrication of enzyme electrodes with a polythiophene derivative and application of them to a glucose fuel cell", SYNTHETIC METALS, vol. 159, no. 17-18, 1 septembre 2009 (2009-09-01), pages 1859-1864, XP026564940, ELSEVIER SEQUOIA, LAUSANNE, CH ISSN: 0379-6779, DOI: 10.1016/J.SYNTHMET. 2009.06.007 [extrait le 2009-07-01]**
- **P. CINQUIN AND AL: "A glucose biofuel cell implanted in rats", PLOSONE, vol. 5, no. 5, E10476, 1 mai 2010 (2010-05-01), pages 1-7, XP002600795, DOI: 10.1371/journal.pone. 0010476**

**Description**

Domaine de l'invention

La présente invention concerne des biopiles (en anglais biofuel cells), de type à sucre-oxygène, par exemple à glucose-oxygène, c'est-à-dire des piles à combustible adaptées à convertir en électricité une partie de l'énergie disponible dans un substrat biodégradable. Une biopile selon le préambule de la revendication 1 est décrite dans l'article de S. Cosnier et al paru dans Electrochemistry Communications, Vol. 12, N° 2, pp. 266-269.

Exposé de l'art antérieur

Divers types de biopiles à glucose-oxygène sont décrits dans l'art antérieur, par exemple dans la demande de brevet PCT/FR2009/050639 (B8606). Dans ces biopiles connues, chaque électrode, anode et cathode, correspond à une enceinte contenant un milieu liquide dans lequel plonge un fil d'électrode. Les enceintes d'anode et de cathode sont délimitées par des membranes pouvant être traversées par l'hydrogène et l'oxygène mais évitant la circulation d'autres éléments plus lourds.

L'anode comprend dans une solution une enzyme et un médiateur redox. L'enzyme est apte à catalyser l'oxydation du sucre et est par exemple choisie dans le groupe comprenant glucose-oxydase si le sucre est du glucose et lactose-oxydase si le sucre est du lactose. Le médiateur redox a un potentiel redox bas susceptible d'échanger des électrons avec l'enzyme d'anode et est par exemple choisi dans le groupe comprenant : ubiquinone (UQ) et ferrocène.

La cathode comprend également dans une solution une enzyme et un médiateur redox. L'enzyme est apte à catalyser la réduction de l'oxygène et est par exemple choisie dans le groupe comprenant : polyphénol oxydase (PPO), laccase et bilirubine oxydase. Le médiateur redox a un potentiel redox haut susceptible d'échanger des électrons avec l'enzyme de cathode et est par exemple choisi dans le groupe comprenant : hydroquinone (QHD) et 2,2'-azinobis-(3-éthylbenzo-thiazoline-6-sulfonate) (ABTS).

Il se produit alors au niveau de l'anode et de la cathode des réactions du type suivant :

$$\text{Cathode :} \quad QH_2 + 1/2\ O_2 \xrightarrow{\text{PPO}} Q + H_2O$$

$$\text{Anode :} \quad \text{glucose} + UQ \xrightarrow{\text{GOX}} \text{gluconolactone} + UQH_2$$

Cathode : $Q + 2H^+ + 2e^- \rightarrow QH_2$
Anode : $UQH_2 \rightarrow UQ + 2H^+ + 2e^-$

ces réactions étant données dans le cas particulier où le sucre est du glucose, l'enzyme d'anode est de la glucose-oxydase (GOX), le médiateur redox d'anode est de l'ubiquinone (UQ), l'enzyme de cathode est de la polyphénol oxydase (PPO), et le médiateur redox de cathode est de la quinhydrone ($QH_2$). On obtient alors un potentiel d'anode de 20 mV et un potentiel de cathode de 250 mV, ce qui conduit à une différence de potentiel à courant nul de la biopile de 230 mV.

De telles biopiles fonctionnent convenablement mais, notamment en ce qui concerne la biopile décrite dans la demande de brevet PCT/FR2009/050639, nécessitent que des conducteurs d'anode et de cathode trempent dans des enceintes contenant des liquides appropriés, ce qui constitue un inconvénient pratique dans de nombreux cas et rend notamment très difficile sinon impossible d'implanter de telles biopiles dans un être vivant.

En effet, on cherche à implanter de telles biopiles dans des êtres vivants, notamment pour alimenter divers actionneurs, tels que des stimulateurs cardiaques, des sphincters artificiels, ou même des coeurs artificiels.

On a proposé des biopiles à électrodes solides. Toutefois, des biopiles utilisant de telles électrodes, notamment quand elles sont implantées dans un être vivant, ont présenté une faible durée de vie.

Résumé

Ainsi, selon un mode de réalisation de la présente invention, on cherche à réaliser des biopiles simples à manipuler et en particulier implantables dans un être vivant, animal ou humain.

Plus particulièrement, un mode de réalisation de la présente invention prévoit une biopile destinée à être immergée dans un milieu liquide contenant un sucre et de l'oxygène, dans laquelle l'anode comprend une enzyme apte à catalyser l'oxydation du sucre et un médiateur redox de potentiel redox bas susceptible d'échanger des électrons avec l'enzyme d'anode et la cathode comprend une enzyme apte à catalyser la réduction de l'oxygène et un médiateur redox de potentiel redox haut susceptible d'échanger des électrons avec l'enzyme de cathode, dans laquelle chacune des électrodes d'anode et de cathode est constituée d'un aggloméré solide d'un matériau conducteur mélangé à l'enzyme et au médiateur redox appropriés, et est solidaire d'un fil d'électrode ; et les électrodes d'anode et de cathode sont entourées d'une membrane semi-perméable laissant passer l'oxygène et le glucose et ne laissant pas passer l'enzyme et le médiateur redox. Dans cette biopile, l'ensemble des électrodes d'anode et de cathode est entouré d'une membrane semi-perméable laissant passer le glucose et l'oxygène et étanche aux enzymes et aux médiateurs redox.

Selon un mode de réalisation de la présente invention, les membranes sont du type membrane de dialyse.

Selon un mode de réalisation de la présente invention, le médiateur à la cathode est choisi dans le groupe comprenant quinone, ABTS, osmocène, ruthénocène, tétraphénylporphyrine de cobalt II, phtalocyanine de zinc.

Selon un mode de réalisation de la présente invention, l'enzyme à la cathode est choisie dans le groupe comprenant polyphénol oxydase (PPO), laccase et bilirubine oxydase.

Selon un mode de réalisation de la présente invention, le médiateur à l'anode est choisi dans le groupe comprenant ubiquinone, ferrocène, cobaltocène, N-méthylphénothiazine, hydrate d'acide 8-hydroxyquinoline-5-sulfonique (HQS).

Selon un mode de réalisation de la présente invention, l'enzyme à l'anode est choisie dans le groupe comprenant glucose-oxydase, lactose-oxydase, galactose oxydase, fructose oxydase, en fonction du sucre à convertir.

Selon un mode de réalisation de la présente invention, le matériau conducteur est du graphite.

Selon un mode de réalisation de la présente invention, le matériau conducteur est un polymère conducteur.

Un autre mode de réalisation de la présente invention prévoit une procédé de fabrication d'une biopile, selon lequel l'anode et la cathode sont formées par compression d'un mélange en solution comprenant un conducteur associé à une enzyme et un médiateur redox appropriés.

Brève description des dessins

Ces objets, caractéristiques et avantages, ainsi que d'autres seront exposés en détail dans la description suivante de modes de réalisation particuliers faite à titre non-limitatif en relation avec les figures jointes parmi lesquelles :

la figure 1 représente très schématiquement une biopile à électrodes solides ; et
la figure 2 représente très schématiquement une biopile selon un mode de réalisation de la présente invention.

Description détaillée

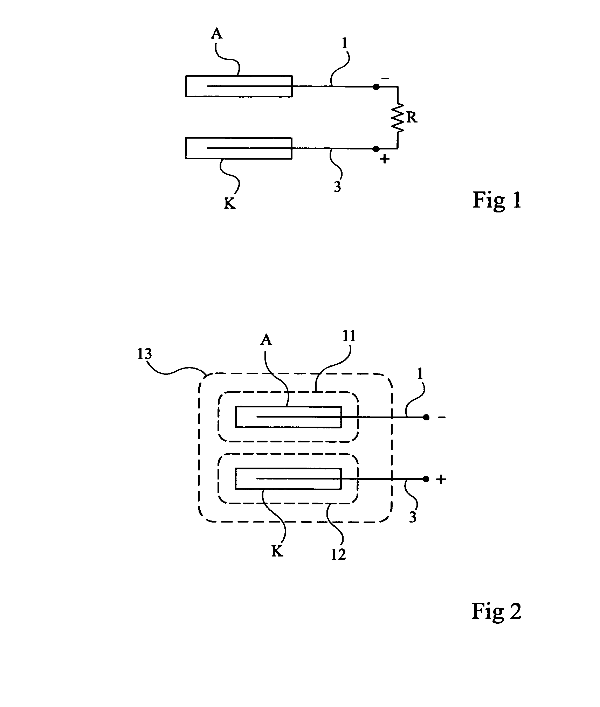

La figure 1 représente une biopile connectée sur une charge R. Cette biopile comprend un corps d'anode A et un corps de cathode K. Le corps d'anode est constitué d'un corps solide comprenant un matériau conducteur associé à une enzyme et à un médiateur redox d'anode appropriés. Le corps d'anode est solidaire d'un fil d'anode 1. De même, l'anode est constituée d'un corps solide formé d'un conducteur associé à une enzyme et à un médiateur de cathode appropriés. Le corps de cathode est solidaire d'un fil de cathode 3. Les fils d'anode et de cathode, par exemple en platine, sont représentés comme pénétrant dans les corps d'anode et de cathode ; ils pourront simplement être collés à ces corps.

Le corps d'anode et le corps de cathode sont de préférence formés par compression d'un matériau conducteur pulvérulent tel que du graphite mélangé à l'enzyme et au médiateur redox appropriés. On pourra aussi utiliser une poudre d'un polymère conducteur tel que polyaniline, polypropylène, polyfluorure de vinylidène.

A titre d'exemple, une anode a été préparée en mélangeant 350 mg de particules de graphite, 110 mg d'UQ, 0,5 ml d'eau et de glycérol (50 $\mu$l) dans un mortier de céramique. Ensuite, 7 mg de GOX et 3 mg de catalase solubilisés dans 100 $\mu$l d'eau ont été incorporés à 400 mg du mélange précédent et mélangés à la main. Une cathode a été préparée de façon similaire : 350 mg de particules de graphite, 170 mg d'hydroquinone, 0,3 ml d'eau et 25 $\mu$l de glycérol ont été mélangés dans un mortier de céramique. Ensuite, 4,5 mg de PPO solubilisés dans 100 $\mu$l d'eau ont été incorporés à 400 mg du mélange précédent et mélangés à la main. Les pâtes résultantes graphite-enzyme-médiateur redox ont été comprimées à une pression de 10000 kg/cm$^2$ pour former des disques. La surface et l'épaisseur des disques étaient respectivement de 1,33 cm$^2$ et de 0,1 cm respectivement. Un fil de platine a été fixé par une colle conductrice au carbone d'un côté de chaque disque et recouvert d'un film de silicium pour renforcer la solidité mécanique du biorevêtement et le contact électrique.

Pour fonctionner en pile, ces corps d'anode et de cathode sont disposés dans un fluide contenant de l'oxygène et un sucre, par exemple du glucose. Les corps d'anode et de cathode peuvent par exemple être implantés à l'intérieur d'un corps animal ou humain, de nombreux emplacements du corps contenant des fluides contenant du glucose et de l'oxygène.

Toutefois, il s'est avéré que des biopiles utilisant de tels corps d'anode et de cathode présentaient une faible durée de vie. Les inventeurs ont attribué ce problème à ce que du matériau redox et/ou de l'enzyme fuient au cours du temps du corps d'anode et du corps de cathode. Pour résoudre ce problème, comme l'illustre la figure 2, chacun des corps d'anode et de cathode est entouré d'une membrane micro-perforée, respectivement 11, 12, telle que des membranes couramment utilisées en dialyse, qui laisse passer le glucose et l'oxygène et interdit le passage de l'enzyme et du médiateur redox de plus fort poids moléculaire. L'ensemble des électrodes d'anode et de cathode peut être entouré d'une membrane semi-perméable 13 laissant passer le glucose et l'oxygène et étanche aux enzymes et aux médiateurs redox, notamment pour éviter que les membranes d'anode et de cathode ne s'obstruent, notamment en cas d'implantation dans un corps animal ou humain.

Des biopiles à glucose du type ci-dessus ont été implantées en position latérale gauche dans les cavités rétro-péritoniale de deux rats pesant respectivement 514 et 512 g. Après stabilisation du potentiel en circuit ouvert, des cycles de charge et décharge ont été réalisés pendant une période de 12 heures. Des courants de décharges de 10 à 50 microampères ont été constatés.

Divers exemples de réalisation avec diverses variantes ont été décrits ci-dessus. On notera que l'homme de l'art pourra combiner divers éléments de ces divers exemples de réalisation et variantes sans faire preuve d'activité inventive.

## Revendications

1. Biopile destinée à être immergée dans un milieu liquide contenant un sucre et de l'oxygène, dans laquelle l'anode comprend une enzyme apte à catalyser l'oxydation du sucre et un médiateur redox de potentiel redox bas susceptible d'échanger des électrons avec l'enzyme d'anode et la cathode comprend une enzyme apte à catalyser la réduction de l'oxygène et un médiateur redox de potentiel redox haut susceptible d'échanger des électrons avec l'enzyme de cathode, dans laquelle

    chacune des électrodes d'anode et de cathode est constituée d'un aggloméré solide d'un matériau conducteur mélangé à l'enzyme et au médiateur redox appropriés, et est solidaire d'un fil d'électrode ; et

    les électrodes d'anode et de cathode sont entourées d'une membrane semi-perméable laissant passer l'oxygène et le glucose et ne laissant pas passer l'enzyme et le médiateur redox,

    **caractérisée en ce que** l'ensemble des électrodes d'anode et de cathode est entouré d'une membrane semi-perméable laissant passer le glucose et l'oxygène et ne laissant pas passer les enzymes et les médiateurs redox.

2. Biopile selon la revendication 1, dans laquelle lesdites membranes sont du type membrane de dialyse.

3. Biopile selon la revendication 1 ou 2, dans laquelle le médiateur à la cathode est choisi dans le groupe comprenant quinone, ABTS, osmocène, ruthénocène, tétraphénylporphyrine de cobalt II, phtalocyanine de zinc.

4. Biopile selon l'une quelconque des revendications 1 à 3, dans laquelle l'enzyme à la cathode est choisie dans le groupe comprenant polyphénol oxydase (PPO), laccase et bilirubine oxydase.

5. Biopile selon l'une quelconque des revendications 1 à 4, dans laquelle le médiateur à l'anode est choisi dans le groupe comprenant ubiquinone, ferrocène, cobaltocène, N-méthylphénothiazine, hydrate d'acide 8-hydroxyquinoline-5-sulfonique (HQS).

6. Biopile selon l'une quelconque des revendications 1 à 5, dans laquelle l'enzyme à l'anode est choisie dans le groupe comprenant glucose-oxydase, lactose-oxydase, galactose oxydase, fructose oxydase, en fonction du sucre à convertir.

7. Biopile selon l'une quelconque des revendications 1 à 6, dans laquelle le matériau conducteur est du graphite.

8. Biopile selon l'une quelconque des revendications 1 à 6, dans laquelle le matériau conducteur est un polymère conducteur.

## Claims

1. A biofuel cell intended to be immersed into a liquid medium containing a sugar and oxygen, wherein the anode comprises an enzyme capable of catalyzing the oxidation of the sugar and a redox mediator of low redox potential capable of exchanging electrons with the anode enzyme, and the cathode comprises an enzyme capable of catalyzing the reduction of oxygen and a redox mediator of high redox potential capable of exchanging electrons with the cathode enzyme, wherein

    each of the anode and cathode electrodes is formed of a solid agglomerate of a conductive material mixed with the appropriate enzyme and redox mediator and is solid with an electrode wire; and

the anode and cathode electrodes are surrounded with a semipermeable membrane letting through oxygen and glucose and blocking the enzyme and the redox mediator,
**characterized in that** the anode and cathode electrode assembly is surrounded with a semipermeable membrane letting through glucose and oxygen and blocking enzymes and redox mediators.

2. The biofuel cell of claim 1, wherein said membranes are dialysis-type membranes.

3. The biofuel cell of claim 1 or 2, wherein the cathode mediator is selected from the group comprising quinone, ABTS, osmocene, ruthenocene, cobalt (II) tetraphenylporphyrin, and zinc phthalocyanine.

4. The biofuel cell of any of claims 1 to 3, wherein the cathode enzyme is selected from the group comprising polyphenol oxidase (PPO), laccase, and bilirubin oxidase.

5. The biofuel cell of any of claims 1 to 4, wherein the anode mediator is selected from the group comprising ubiquinone, ferrocene, cobaltocene, N-methylphenothiazine, and 8-hydroxyquinoline-5-sulfonic acid hydrate (HQS).

6. The biofuel cell of any of claims 1 to 5, wherein the anode enzyme is selected from the group comprising glucose oxidase, lactose oxidase, galactose oxidase, and fructose oxidase, according to the sugar to be converted.

7. The biofuel cell of any of claims 1 to 6, wherein the conductive material is graphite.

8. The biofuel cell of any of claims 1 to 6, wherein the conductive material is a conductive polymer.

**Patentansprüche**

1. Eine Biobrennstoffzelle, die dafür bestimmt ist, in ein flüssiges Medium, welches einen Zucker und Sauerstoff enthält, eingetaucht zu werden, wobei die Anode Folgendes aufweist: ein Enzym, das in der Lage ist, die Oxydation des Zuckers zu beschleunigen bzw. zu katalysieren, und einen Redoxmediator bzw. Redoxvermittler mit niedrigem Redoxpotential, welcher in der Lage ist, Elektronen mit dem Anoden-Enzym auszutauschen, und wobei die Kathode ein Enzym aufweist, welches in der Lage ist, die Reduktion des Sauerstoffs zu katalysieren, und wobei der Redoxvermittler mit hohem Redoxpotential in der Lage ist, Elektronen mit dem Kathoden-Enzym auszutauschen, wobei jede der Anoden- und Kathodenelektroden aus einem festen Agglomerat eines leitenden Materials hergestellt ist, welches mit dem geeigneten Enzym und Redoxvermittler vermischt ist, und fest mit einem Elektrodendraht verbunden ist; und
wobei die Anoden- und Kathodenelektroden von einer semipermeablen Membran umgeben sind, welche Sauerstoff und Glukose hindurch lässt und das Enzym und den Redoxvermittler blockiert,
**dadurch gekennzeichnet, dass** die Anoden- und Kathodenelektrodenanordnung von einer semipermeablen Membran umgeben ist, welche Glukose und Sauerstoff hindurch lässt und Enzyme und Redoxvermittler blockiert.

2. Biobrennstoffzelle nach Anspruch 1, wobei die Membranen Membranen von der Dialyse-Bauart sind.

3. Biobrennstoffzelle nach Anspruch 1 oder 2, wobei der Kathoden-Mediator bzw. Kathoden-Vermittler aus der Gruppe ausgewählt ist, welche aus Chinon, ABTS, Osmocen, Ruthenocen, Kobalt(II)-tetraphenylporphyrin und Zink-Phtalocyanin besteht.

4. Biobrennstoffzelle nach einem der Ansprüche 1 bis 3, wobei das Kathoden-Enzym aus der Gruppe ausgewählt ist, die aus Polyphenoloxidase (PPO), Laccase und Bilirubinoxidase besteht.

5. Biobrennstoffzelle nach einem der Ansprüche 1 bis 4, wobei der Anoden-Vermittler aus der Gruppe ausgewählt ist, welche aus Ubichinon, Ferrocen, Cobaltocen, N-Methyl-phenothiazin und 8-Hydroychinolin-5-sulfonsäurehydrat (HQS) besteht.

6. Biobrennstoffzelle nach einem der Ansprüche 1 bis 5, wobei das Anoden-Enzym aus der Gruppe ausgewählt ist, die aus Glukose-Oxidase, Laktose-Oxidase, Galaktose-Oxidase und Fruktose-Oxidase besteht, abhängig von dem Zucker der konvertiert werden soll.

7. Biobrennstoffzelle nach einem der Ansprüche 1 bis 6, wobei das leitende Material Graphit ist.

**8.** Biobrennstoffzelle nach einem der Ansprüche 1 bis 6, wobei das leitende Material ein leitendes Polymer ist.

Fig 1

Fig 2

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2009050639 W

**Littérature non-brevet citée dans la description**

- **S. COSNIER et al.** *Electrochemistry Communications,* vol. 12 (2), 266-269